# EUROPÄISCHE PATENTSCHRIFT

(11) **EP 0 804 279 B1**
(45) Veröffentlichungstag und Bekanntmachung des Hinweises auf die Patenterteilung: **12.12.2001**
(21) Anmeldenummer: 95932759.4
(22) Anmeldetag: 15.09.1995
(51) Int. Cl.: B01F 17/00, C07C 233/38, C07C 305/10, C11D 1/52, C11D 1/88, C11D 1/92

(54) **AMPHIPHILE VERBINDUNGEN MIT MINDESTENS ZWEI HYDROPHILEN UND MINDESTENS ZWEI HYDROPHOBEN GRUPPEN AUF DER BASIS VON AMIDEN**
AMPHIPHILIC COMPOUNDS WITH AT LEAST TWO HYDROPHILIC AND AT LEAST TWO HYDROPHOBIC GROUPS BASED ON AMIDES
COMPOSES AMPHIPHILES COMPORTANT AU MOINS DEUX GROUPES HYDROPHILES ET AU MOINS DEUX GROUPES HYDROPHOBES A BASE D'AMIDES

(30) Priorität: 11.11.1994 DE 4440328
(43) Veröffentlichungstag der Anmeldung: 05.11.1997
(73) Patentinhaber: SASOL Germany GmbH, 22297 Hamburg (DE)
(72) Erfinder: KWETKAT, Klaus, D-59192 Bergkamen (DE); RUBACK, Wulf, D-48249 Dülmen (DE)
(74) Vertreter: Schupfner, Gerhard D., Dr. Dipl.-Chem.
(86) Internationale Anmeldenummer: EP9503635
(87) Internationale Veröffentlichungsnummer: WO9614926

(56) Entgegenhaltungen:
- EP-A- 0 258 923
- WO-A-95/11288
- WO-A-95/19955
- DE-A- 4 227 894
- DATABASE WPI Section Ch, Week 8528 Derwent Publications Ltd., London, GB; Class D25, AN 85-167924 & JP,A,60 096 695 ( SANYO CHEM IND LTD) , 30.Mai 1985

## Beschreibung

Die Erfindung betrifft amphiphile Verbindungen mit mindestens zwei hydrophilen und mindestens zwei hydrophoben Gruppen auf der Basis von Amiden.

Als amphiphile Substanzen sind eine große Vielfalt an anionischen, kationischen, nichtionischen und zwitterionischen Verbindungen bekannt. Die weitaus meisten dieser Substanzen bestehen aus einer hydrophilen Kopfgruppe und wenigstens einem hydrophoben Teil.

Bei den amphiphilen Substanzen gibt es aus ökologischen Gründen, z. B. bezüglich der Verringerung des Verpackungs- und Transportaufwandes, die Notwendigkeit, immer größere Wirkung pro Masse an eingesetzter Substanz zu erzielen. Da eine Optimierung durch Mischung von amphiphilen Substanzen nur sehr begrenzt weiterführt, sind neue amphiphile Substanzen mit einem höheren Wirkungsgrad erforderlich. Es müssen daher insbesondere Stoffe mit niedrigeren kritischen Micellbildungskonzentrationen und/oder niedrigeren Ober- und Grenzflächenspannungen gefunden werden, um die Einsatzmengen an Wirksubstanz deutlich reduzieren zu können.

Erste Lösungsansätze in dieser Richtung durch Verdoppelung eines Teils der Struktur (hydrophile Kopfgruppe, hydrophobe Gruppe) sind bereits bekannt. So können kationische grenzflächenaktive Verbindungen durch die Addition von langkettigen Alkylhalogeniden an permethylierte Alkylendiamine erhalten werden [R. Zana, M. Benrraou, R. Rueff, Langmuir, 7 (1991) 1072; R. Zana, Y. Talmon, Nature, 362 (1993) 228; E. Alami, G. Beinert, P. Marie, R. Zana, Langmuir, 9 (1993) 1465].

Anionische grenzflächenaktive Verbindungen mit wenigstens zwei hydrophilen und wenigstens zwei hydrophoben Gruppen sind bisher nur auf der Basis von Diglycidylethern hergestellt worden (US 5 160 450, JP 01 304 033, JP 4 124 165). Diglycidylether gelten jedoch als toxikologisch bedenklich und sind recht teuer. Darüber hinaus wird für ihre Herstellung Epichlorhydrin verwendet, was zu großen Mengen an Reststoffen führt, so daß diese Verbindungen unter ökotoxikologischen wie auch ökonomischen Gesichtspunkten nicht mehr zeitgemäß sind.

Es bestand daher die Aufgabe, amphiphile Verbindungen aufzufinden, die wenigstens zwei hydrophile und wenigstens zwei hydrophobe Gruppen aufweisen, wobei die amphiphilen Verbindungen einen sehr hohen Wirkungsgrad, bezogen auf die Einsatzmenge, haben, und die darüber hinaus aus technisch leicht verfügbaren Rohstoffen ohne großen Anfall von unerwünschten Nebenprodukten hergestellt werden können.

Die Aufgabe wird erfindungsgemäß durch anionische amphiphile Di- oder Oligoamide, deren Grundkörper aus Di- oder Oligoaminen und Fettsäuren bzw. Fettsäuremethylestern hergestellt werden können, gelöst. Die entsprechenden Di- oder Oligoamide können alkoxyliert werden. Aus den nichtionischen amphiphilen Verbindungen sind die anionischen amphiphilen Verbindungen enhältlich, indem man z. B. die vorgenannten Verbindungen mit SO₃/Inertgas (oder Oleum oder Chlorsulfonsäure), mit Polyphosphorsäure, mit einer Halogenessigsäure, mit einem Sulton oder mit einem Taurin, umsetzt und jeweils anschließend neutralisiert.

Bei den erfindungsgemäßen anionischen amphiphilen Verbindungen handelt es sich daher um Verbindungen der allgemeinen Formel I 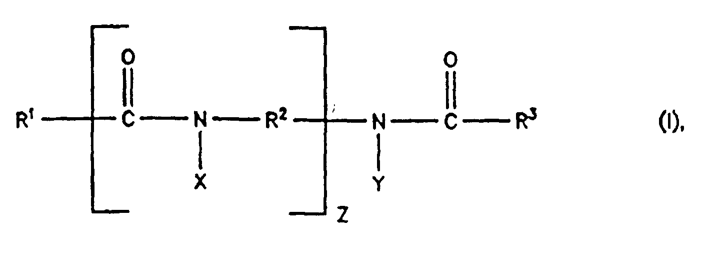 wobei R¹, R², R³, X, Y und Z in der Formel I die im folgenden beschriebenen Bedeutungen haben:
R¹ und R³ stehen unabhängig voneinander für einen unverzweigten oder verzweigten, gesättigten oder ungesättigten Kohlenwasserstoffrest mit 1 bis 22, vorzugsweise 7 bis 17, Kohlenstoffatomen.

Es seien als Substituenten R¹ und R³ im einzelnen die Reste Methyl, Ethyl, n-Propyl, n-Butyl, n-Pentyl, n-Hexyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl, n-Eicosyl, n-Uneicosyl, n-Docosyl und ihre verzweigtkettigen Isomeren sowie die entsprechenden einfach, zweifach oder dreifach ungesättigten Reste genannt.

R² bedeutet einen Spacer, bestehend aus einer unverzweigten oder verzweigten Kette mit 2 bis 100 Kohlenstoffatomen, die je 0 bis 20 Sauerstoff- und Stickstoffatome und 0 bis 4 Schwefelatome und 0 bis 3 Phosphoratome enthält, und die 0 bis 20 funktionelle Seitengruppen, wie z. B. Hydroxyl-, Carbonyl-, Carboxyl-, Amino- und/oder Acylaminogruppen, aufweist.

Der Spacer R² bedeutet insbesondere
◆ als Grundkörper unverzweigte oder verzweigte Alkylenketten

   -CₐH₂ₐ- (II)

   mit a = 2 bis 18, vorzugsweise a = 3 bis 6;
◆ als Grundkörper unverzweigte oder verzweigte Alkenylenketten

   -C_{b}H_{2b}-CH=CH-C_{c}H_{2c}- (III)

   mit b + c = 2 bis 16, wobei b und c jeweils größer als Null sind;
◆ als Grundkörper unverzweigte oder verzweigte Alkinylenketten

   -C_{d}H_{2d}-C≡C-CₑH₂ₑ- (IV)

   mit d + e = 2 bis 16, wobei d und e jeweils größer als Null sind, und wobei bei den Grundkörpern gemäß den Formeln II bis IV der Spacer an einer beliebigen Stelle der Kette zusätzlich 0 bis 4 Carbonyl-, Amino- oder Acylaminogruppen enthält;
◆ Alicyclen gemäß der Formel V

   -C_{f}H_{2f}-cyclo C₆H₁₀-C_{g}H_{2g}- (V)

   mit f und g = je 1 bis 6
   oder gemäß der Formel VI

   -3(4),8(9)-di(methylen)-tricyclo[5.2.1.0^{2.6}]decan- (VI);
◆ unsubstituierte oder substituierte Aromaten gemäß der Formel VII

   -CₕH₂ₕ-C₆R₄-(CᵢH₂ᵢ-C₆R₄)_{j₁}-Cⱼ₂H₂ⱼ₂- (VII)

   oder gemäß der Formel VIII

   -CₕH₂ₕ-C₁₀R₆-CⱼH₂ⱼ- (VIII)

   mit h, j, j₁ und j₂ = je 0 bis 8 und i = 1 bis 8 und
   mit R = unabhängig voneinander jeweils H oder C₁- bis C₄-Alkyl;
◆ eine Kette mit funktionellen Seitengruppen, insbesondere eine Amino-, Acylamino-, Carbonyl- oder Carboxylfunktion.

Weiterhin enthält der Spacer R² je 0 bis 20, vorzugsweise 1 bis 12, Sauerstoff- und/oder Stickstoffatome, 0 bis 4 Schwefelatome und 0 bis 3 Phosphoratome, wobei mindestens eines der Heteroatome mindestens einmal vorkommt.

R² hat damit weiterhin insbesondere die Bedeutung
◆ einer Verbindung gemäß der Formel IX

   -CₖH₂ₖ-CₓR_{y}-Z-CₓR_{y}-CₗH₂ₗ- (IX)

   mit k und l = je 0 bis 8, x = 6 und y = 4 oder
   x = 10 und y = 6 oder x = 14 und y = 8, und
   Z = 0, NH, NR¹, N-C(O)R¹, SO₂, wobei R¹ einen Kohlenwasserstoffrest mit 1 bis 22 Kohlenstoffatomen und R unabhängig voneinander jeweils H oder C₁-C₄-Alkyl bedeuten;
◆ einer Verbindung gemäß der Formel X

   -CₘH₂ₘ-(OCₙH₂ₙ)ₚ-C_{q}H_{2q}- (X)

   mit m = 1 bis 4, n = 2 bis 4, p = 1 bis 20, vorzugsweise p = 1 bis 4,
   und q = 1 bis 4,
   wobei auch gemischte Alkoxideinheiten auftreten können und dann die Reihenfolge der Alkoxideinheiten beliebig ist;
◆ einer Verbindung gemäß der Formel XI

   -CᵣH₂ᵣ(RNCₛH₂ₛ)ₜ-CᵤH₂ᵤ- (XI)

   oder gemäß der Formel XII

   -[CᵣH₂ᵣ[RN-C(O)-NR]ₜ-CᵤH₂ᵤ]_{w}- (XII)

   oder gemäß der Formel XIII

   -[CᵣH₂ᵣ[RNC(O)CᵥH₂ᵥC(O)NR]ₜ-CᵤH₂ᵤ]_{w}- (XIII)

   oder gemäß der Formel XIV

   -[CᵣH₂ᵣ[RN-C(O)-CH=CH-C(O)-NR]ₜ-CᵤH₂ᵤ]_{w}- (XIV)

   oder gemäß der Formel XV

   -[CᵣH₂ᵣ[RNC(O)CₓR_{y}C(O)NR]ₜ-CᵤH₂ᵤ]_{w}- (XV)

   mit r = 2 bis 4, s = 2 bis 4, t = 1 bis 20, vorzugsweise t = 1 bis
   u = 2 bis 4, v = 0 bis 12, w = 1 bis 6, x = 6 und y = 4 oder
   x = 10 und y = 6 oder
   x = 14 und y = 8
   mit R = unabhängig voneinander jeweils H oder C₁- bis C₄-Alkyl,

X und Y stehen unabhängig voneinander für Substituenten der Formel XVI

-(C₂H₄O)_{α}(C₃H₆O)_{β}H (XVI)

mit α = 0 bis 50, vorzugsweise α = 10 bis 30,
β = 0 bis 60, vorzugsweise β = 20 bis 40,
und α + β = 1 bis 100, vorzugsweise α + β = 10 bis 50,
wobei R² nicht C₂H₄ ist, wenn β = 0 ist,
oder für Substituenten der Formel XVII

-(C₂H₄O)_{γ}(C₃H₆O)_{δ}-FR (XVII)

mit jeweils γ = 0 bis 20, vorzugsweise γ = 0 bis 8,
δ = 0 bis 20, vorzugsweise δ = 0 bis 12,
und γ + δ = 1 bis 40, vorzugsweise γ + δ = 5 bis 20,
wobei FR für einen funktionellen Rest -CH₂-COOM, -SO₃M, -PO(OM)₂,

-C(O)-C₂H₃(SO₃M)-CO₂M'

oder -C₂H₄-SO₃M mit M, M' = Alkali, Ammonium-, Alkanolammonium oder ½ Erdalkali steht
und wobei die Alkoxideinheiten statistisch oder blockweise eingebunden sind und die Reihenfolge beliebig ist.

Der Oligomerisierungsgrad Z beträgt 1 bis 10, vorzugsweise Z = 1 bis 4, besonders bevorzugt Z = 1.

Der Alkoxylierungsgrad ist jeweils ein Mittelwert und kann jeden beliebigen, auch nicht ganzzahligen Wert in den angegebenen Grenzen einnehmen.

Die erfindungsgemäßen amphiphilen Verbindungen zeichnen sich meist durch extrem niedrige kritische Micellbildungskonzentrationen (CMC) und sehr niedrige Oberflächen- und Grenzflächenspannungen (z. B. gegen Paraffin) aus, was auf ihre besondere Struktur - wenigstens zwei hydrophile Gruppen und wenigstens zwei hydrophobe Gruppen - zurückgeführt werden muß. Darüber hinaus weisen die meisten von ihnen ein recht hohes hydrophiles Suspendiervermögen auf, das etwa auf halbem Wege zwischen dem konventioneller Tenside und dem des Pentanatriumtripolyphosphats liegt. Einige dieser Verbindungen sind extrem schnelle Netzmittel.

Die amphiphilen Verbindungen gemäß dieser Erfindung eignen sich insbesondere als Emulgatoren, Demulgatoren, Detergenzien, Dispergatoren und Hydrotropica in Industrie und Haushalt, beispielsweise auf den Gebieten Metallbearbeitung, Erzgewinnung, Oberflächenveredelung, Waschen und Reinigen, Kosmetik, Medizin und Nahrungsmittelverarbeitung und -zubereitung.

Hierbei können sie mit allen gängigen anionischen, nichtionischen, kationischen und ampholytischen grenzflächenaktiven Substanzen kombiniert werden. Als Beispiele für nichtionische grenzflächenaktive Substanzen, die für eine Kombination eingesetzt werden können, seien genannt: Fettsäureglyceride, Fettsäurepolyglyceride, Fettsäureester, Ethoxylate höherer Alkohole, Polyoxyethylenfettsäureglyceride, Polyoxyethylenpropylenglykolfettsäureester, Polyoxyethylensorbitanfettsäureester, Polyoxyethylen-Rhizinusöl- oder gehärtete Rhizinusöl-Derivate,Polyoxyethylenlanolinderivate,Polyoxyethylenfettsäureamide, Polyoxyethylenalkylamine, Alkanolamine, Alkylaminoxide, Derivate von Eiweißhydrolysaten, Hydroxymischether, Alkylpolyglycoside und Alkylglucamide.

Als Beispiele für anionische grenzflächenaktive Substanzen, die für Kombinationen eingesetzt werden können, seien genannt: Seifen, Ethercarbonsäuren und deren Salze, Alkylsulfonate, α-Olefinsulfonate, Sulfonate höherer Fettsäureester, höhere Alkoholsulfate, Alkoholethersulfate, Hydroxymischethersulfate, Salze von Phosphatestern, Tauride, Isethionate, lineare Alkylbenzolsulfonate, Cumolsulfonat, Alkylarylsulfonate, Sulfate der Polyoxyethylenfettsäureamide und Salze von Acylaminosäuren.

Als Beispiele für kationische gängige grenzflächenaktive Substanzen, die für Kombinationen eingesetzt werden können, seien genannt: Alkyltrimethylammoniumsalze, Dialkyldimethylammoniumsalze, Alkyldimethylbenzylammoniumsalze, Alkylpyridiniumsalze, Alkylisochinoliniumsalze, Benzethoniumchloride und kationische Acylaminosäurederivate.

Als Beispiele für ampholytische grenzflächenaktive Substanzen, die für Kombinationen eingesetzt werden können, seien genannt: Aminosäuren, Betaine, Sulfobetaine, Imidazolinderivate, Sojaöllipide und Lecithin.

Darüber hinaus können die erfindungsgemäßen amphiphilen Verbindungen auch für sich miteinander kombiniert werden.

Den erfindungsgemäßen amphiphilen Verbindungen können ebenfalls gängige Additive zugesetzt werden. Solche Additive werden speziell für eine Formulierung ausgewählt und umfassen üblicherweise anorganische Salze, wie Natriumchlorid und -sulfat, sowie Builder, Hydrotropica, UV-Absorber, Weichmacher, Chelatbildner, Viskositätsmodifizierer und Riechstoffe.

Die oben genannten Verbindungen lassen sich nach bekannten Methoden herstellen: Die Di- oder Oligoamine werden mit je einem Äquivalent Fettsäure oder Fettsäuemethylester pro freier NH-Funktion bei erhöhten Temperaturen (80 bis 180 °C) optional in Gegenwart eines Katalysators umgesetzt, wobei das entstehende Wasser oder das Methanol unter Vakuum entfernt wird. Anschließend wird bei Temperaturen von 130 bis 190 °C in Gegenwart eines basischen Katalysators alkoxyliert. Die flüssigen oder bei niedrigen Temperaturen erweichenden Produkte können anschließend mit SO₃/Inertgas (Oleum oder Chlorsulfonsäure) oder Polyphosphorsäure oder mit einer Halogenessigsäure, einem Sulton oder mit Isethionsäure umgesetzt und mit wäßrigen Alkali- oder Erdalkalihydroxiden oder wäßrigem Ammoniak oder Alkanolaminen neutralisiert werden. Bei Bedarf werden die Produkte in wäßriger Lösung mit Wasserstoffperoxid (0,1 bis 2,0 %, bezogen auf Feststoff) gebleicht.

### Beispiele

Folgende Beispiele sollen die Erfindung erläutern, sie jedoch nicht darauf einschränken. Alle Angaben in Prozent sind Gewichtsprozente.

### Beispiel 1:

R¹ = R³ = -C₇H₁₅/-C₉H₁₉ (1 : 1), R² = -C₂H₄-, X,Y = -(C₂H₄O)₅SO₃Na In einem 1 l-Vierhalskolben mit Rührer, Thermometer, Wasserauskreiser und angeschlossenem Rückflußkühler werden 91,8 g Ethylendiamin mit 441,5 g Octan- und Decansäuremethylestergemisch (1 : 1) gemischt und auf 120 bis 140 °C erhitzt. Innerhalb von 7 Stunden werden 72,3 ml Methanol entfernt und die Reaktion anschließend beendet. Die Reinheit der drei aus dem Homologengemisch entstandenen Diamide wird mittels ¹³C-NMR überprüft und liegt bei > 99 mol-%, Ausbeute 447,2 g (85 % d. Th.).

170,0 g des Diamidgemisches werden mit 150 ml Testbenzin gemischt und mit 1,1 g festen Kaliumhydroxids versetzt. Bei 160 °C wird das Gemisch mit 220 g Ethylenoxid innerhalb von 6 Stunden umgesetzt. Der Katalysator wird mit Milchsäure neutralisiert und das ausfallende Kaliumsalz sowie eventuell nicht umgesetztes Diamid abfiltriert. Anhand der Auswaage wird auf kompletten Umsatz des Ethylenoxids geprüft und per ¹³C-NMR das Verhältnis Ethylenoxid zum Restmolekül bestimmt: 10 EO-Einheiten pro Restmolekül, Ausbeute: 390 g (quantitativ), keine verbleibenden N-H - Funktionen im Produktgemisch laut IR.

Zu einem Gemisch aus 117,7 g Chlorsulfonsäure und 60,2 g Essigsäure werden 218,5 g des erhaltenen Ethoxylates in 300 ml Dichlormethan so zugetropft, daß die Temperatur 5 °C nicht übersteigt. Anschließend wird bei Raumtemperatur 3 Stunden gerührt (laut Kontrolle per Dünnschichtchromatographie ist das Ethoxylat vollständig umgesetzt). Es wird mit 2-normaler Natriumcarbonatlösung neutralisiert und mit gesättigter Natriumhydrogencarbonatlösung verdünnt. Mit n-Butanol wird das Produkt extrahiert und anschließend der Alkohol entfernt.
Die Reinheitskontrolle erfolgt per Dünnschichtchromatographie und NMR. Ausbeute: 96,8 g (36 % d. Th.), Reinheit: 90 %; charakteristische ¹³C-NMR Daten (DMSO-D₆): 171,8 ppm, 69,2 ppm, 68,7 ppm, 67,8 ppm, 64,6 ppm 62,3 ppm, 32,9 ppm, 30,7 ppm, 27,9-28,3 ppm, 24 ppm (breit), 21,9 ppm, 13,1 ppm;
CMC: 0,011 g/l, γ_{CMC} = 30 mN/m (in VE-Wasser, 20 °C), γ_{Paraffin} = 2,5 mN/m (0,1 g/l WAS in VE-Wasser, 20 °C).

### Beispiel 2:

R¹ = R³ = -C₇H₁₅/-C₉H₁₉ (1 : 1), R² = -C₂H₄-, X,Y = -(C₂H₄O)₁₀SO₃Na
Die Reaktion wird analog zu Beispiel 1 durchgeführt:
Es wird dasselbe Diamidgemisch wie in Beispiel 1 hergestellt.

Ethoxylierung: 170 g Diamidgemisch, 150 ml Testbenzin, 440 g Ethylenoxid, 1,1 g KOH, laut NMR 20 EO-Einheiten pro Restmolekül, Ausbeute: 610 g (quantitativ).

### Sulfatierung:

217,5 g Ethoxylat, 78,8 g Chlorsulfonsäure, 40,3 g Essigsäure,
Ausbeute: 123,7 g (50 % d. Th.), Reinheit: 90 %, charakteristische ¹³C-NMR Daten (DMSO-D₆): 172,0 ppm, 69,4 ppm, 69,1 ppm, 65,2 ppm, 28,5 ppm (breit), 21,1 ppm, 13,4 ppm; CMC: 0,13 g/l, γ_{CMC} = 33 mN/m (VE-Wasser, 20 °C),
γ_{Paraff in} = 15,5 mN/m (0,1 g/l WAS in VE-Wasser, 20 °C).

### Beispiel 3:

R¹ = R³ = -C₇H₁₅/-C₉H₁₉ (1 : 1), R² = -C₆H₁₂-, X,Y = -(C₂H₄O)₈SO₃Na
Die Reaktion wird analog zu Beispiel 1 durchgeführt:
116,2 g Hexamethylendiamin, 344,0 g Octan- und Decansäuregemisch (1 : 1), 0,2 g Zn0,
Reaktionszeit: 7 Stunden, Reaktionstemperatur: 140 bis 145 °C, Ausbeute an Diamidgemisch: 465 g, Reinheit: > 95 %;

Ethoxylierung: 170 g Diamidgemisch, 150 ml Testbenzin, 292 g Ethylenoxid, 1,1 g KOH,
Ausbeute Ethoxylat: 370 g, 8 EO-Einheiten pro Restmolekül;

### Sulfatierung: analog Beispiel 1

Ausbeute: 105,4 g (62 % d. Th.), Reinheit: 92 %, charakteristische
¹³C-NMR Daten (DMSO-D₆): 173,4 ppm (breit), 70,4 ppm, 69,3 ppm, 68,9 ppm, 68,1 ppm, 33,1 ppm, 28 bis 29 ppm (breit), 22,0 ppm, 13,0 ppm; CMC:
0,016 g/l, γ_{CMC} = 28,5 mN/m (in VE-Wasser, 20 °C), γ_{Paraffin} = 3,0 mN/m (0,1 g/l WAS in VE-Wasser, 20 °C).

## Patentansprüche

1. Anionische amphiphile Verbindungen der Formel I 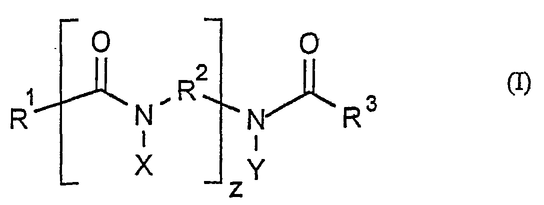 in der
R¹ und R³ unabhängig voneinander einen Kohlenwasserstoffrest mit 1 bis 22 Kohlen-Stoffatomen,
R² einen Spacer,
X und Y unabhängig voneinander funktionelle Gruppen bedeuten und der Oligomerisierungsgrad z = 1 bis 10 beträgt,
wobei X und Y unabhängig voneinander für
- Substituenten der Formel XVI
-(C₂H₄O)_{α}(C₃H₆O)_{β}H (XVI)
mit α = 0 bis 50, β = 0 bis 60, α + β = 1 bis 100 steht, wobei R² nicht C₂H₄ ist, wenn β=o ist;
- oder Substituenten der Formel XVII
-(C₂H₄O)_{γ}(C₃H₆O)_{δ}-FR (XVII)
mit jeweils γ = 0 bis 20 , δ = 0 bis 20, und γ + δ = 1 bis 40, steht,
wobei FR für einen funktionellen Rest -CH₂-COOM, -SO₃M, -P O(OM)₂, -C(O)-C₂H₃(SO₃M)-CO₂M'oder -C₂H₄-SO₃M mit M, M' = Alkali, Ammonium, Alkanolammonium oder 1/2 Erdalkali steht,
wobei die Alkoxideinheiten statistisch oder blockweise eingebunden sind und die Reihenfolge beliebig ist.

2. Verbindungen nach Anspruch 1, **dadurch gekennzeichnet, daß**
die Kohlenwasserstoffreste R¹ und R³ unverzweigt oder verzweigt, gesättigt oder ungesättigt sind und
der Spacer R² eine unverzeigte oder verzweigte Kette mit 2 bis 100 Kohlenstoffatomen, die je 0 bis 20 Sauerstoff- und Stickstoffatome und 0 bis 4 Schwefelatome und 0 bis 3 Phosphoratome enthält und die 0 bis 20 funktionelle Seitengruppen aufweist, bedeutet

3. Verbindungen nach einem der Ansprüche 1 oder 2, **dadurch gekennzeichnet, daß** der Oligomerisierungsgrad z = 1 bis 4 beträgt.

4. Verbindungen nach einem der Ansprüche 1 bis 3, **dadurch gekennzeichnet, daß** die Kohlenwasserstoffreste R¹ und R³ in der Formel I unabhängig voneinander 7 bis 17 Kohlenstoffatome enthalten.

5. Verbindungen nach einem der Ansprüche 1 bis 4, **dadurch gekennzeichnet, daß** R² einen Spacer bedeutet,
der als Grundkörper eine unverzweigte oder verzweigte Alkylenkette der Formel II
-CₐH₂ₐ- (II)
mit a = 2 bis 18,
oder eine unverzweigte oder verzweigte Alkenylenkette der Formel III
-C_{b}H_{2b}-CH=CH-C_{c}H_{2c}- (II)
mit b + c = 2 bis 16, wobei b und c jeweils größer als Null sind,
oder eine unverzweigte oder verzweigte Alkinylenkette der Formel IV
-C_{d}H_{2d}-C ≡ C-CₑH₂ₑ- (IV)
mit d + e = 2 bis 16, wobei d und e jeweils größer als Null sind, und wobei den Grundkörpern gemäß den Formeln II bis IV der Spacer an einer beliebigen Stelle der Kette 0 bis 4 Carbonyl-, Amino- oder Acylaminogruppen aufweist,
enthält.

6. Verbindungen nach einem der Ansprüche 1 bis 4, **dadurch gekennzeichnet, daß** R² einen Spacer bedeutet, der aus Alicyclen gemäß der Formel V
-C_{f}H_{2f}-cyclo C₆H₁₀-C_{g}H_{2g}- (V)
mit f und g = je 1 bis 6,
oder gemäß der Formel VI
-3(4),8(9)-di(methylen)-tricyclo[5.2.1.0^{2.6}]decan- (VI)
besteht.

7. Verbindungen nach einem der Ansprüche 1 bis 4, **dadurch gekennzeichnet, daß** R² einen Spacer bedeutet, der aus unsubstituierten oder substituierten Aromaten gemäß der Formel VII
-CₕH₂ₕ-C₆R₄-(CᵢH₂ᵢ-C₆R₄)ⱼ₁-Cⱼ₂H₂ⱼ₂- (VII)
oder gemäß der Formel VIII
-CₕH₂ₕ-C₁₀R₆-CⱼH₂ⱼ- (VIII)
mit h, j, j1 und j2 = je 0 bis 8 und i = 1 bis 8 und
mit R = unabhängig voneinander jeweils H oder C1- bis C4-Alkyl besteht.

8. Verbindungen nach einem der Ansprüche 1 bis 5, **dadurch gekennzeichnet, daß** der Spacer R² als funktionelle Seitengruppen Amino-, Acylamino-, Carbonyl- oder Carboxylfunktionen trägt.

9. Verbindungen nach einem der Ansprüche 1 bis 5, **dadurch gekennzeichnet, daß** in dem Spacer R² je 0 bis 20 Sauerstoff- und/oder Stickstoffatome, 0 bis 4 Schwefelatome und 0 bis 3 Phosphoratome enthalten sind, wobei mindestens eines der Heteroatome mindestens einmal vorkommt.

10. Verbindungen nach Anspruch 9, **dadurch gekennzeichnet, daß** R² einen Spacer gemäß der Formel (IX)
-CₖH₂ₖ-CₓR_{y}-Z-CₓR_{y}-C₁H₂₁- (IX)
mit k und l = je 0 bis 8, x = 6 und y = 4 oder x = 10 und y = 6 oder x = 14 und y = 8, und Z = O, CO, NH, NR¹, N-C(O)R¹, SO₂, wobei R¹ für einen Kohlenwasserstoffrest mit 1 bis 22 Kohlenstoffatomen und R unabhängig voneinander jeweils für H oder C1- bis C4-Alkyl steht, bedeutet.

11. Verbindungen nach Anspruch 9, **dadurch gekennzeichnet, daß** R² einen Spacer gemäß der Formel X
-CₘH₂ₘ-(CₙH₂ₙO)ₚ-C_{q}H_{2q}- (X)
mit m = 1 bis 4, n = 2 bis 4, p = 1 bis 20 und q = 1 bis 4, wobei auch gemischte Alkoxideinheiten auftreten können und dann die Reihenfolge der Alkoxideinheiten beliebig ist, bedeutet.

12. Verbindungen nach Anspruch 9, **dadurch gekennzeichnet, daß** R² einen Spacer gemäß der Formel XI
-CᵣH₂ᵣ(RNCₛH₂ₛ)ₜ-CᵤH₂ᵤ- (Xi)
oder gemäß der Formel XII
-[CᵣH₂ᵣ[RN-C(O)-NR]ₜ-CᵤH₂ᵤ]_{w}- (XII)
oder gemäß der Formel XIII
-[CᵣH₂ᵣ[RNC(O)CᵥH₂ᵥC(O)NR]ₜ-CᵤH₂ᵤ]_{w}- (XIII)
oder gemäß der Formel XIV
-[CᵣH₂ᵣ[RN-C(O)-CH=CH-C(O)-NR]ₜ-CᵤH₂ᵤ]_{w}- (XIV)
oder gemäß der Formel XV
-[CᵣH₂ᵣ[RNC(O)CₓR_{y}C(O)NR]ₜ-CᵤH₂ᵤ]_{w}- (XV)
mit r = 2 bis 4, s = 2 bis 4, t = 1 bis 20, u= 2 bis 4, v = 0 bis 12, w = 1 bis 6, x = 6 und y = 4 oder x = 10 und y = 6 oder x = 14 und y = 8 mit R = unabhängig voneinander jeweils H oder C1- bis C4-Alkyl, bedeutet.

13. Verwendung der Verbindungen nach einem der Ansprüche 1 bis 12 als Emulgatoren oder Demulgatoren.

14. Verwendung der Verbindungen nach einem der Ansprüche 1 bis 12 als Hilfsmittel bei der Metallbearbeitung, Erzgewinnung oder Oberflächenveredelung.

15. Verwendung der Verbindungen nach einem der Ansprüche 1 bis 12 als Textilhilfsmittel oder für das Reinigen und Waschen von Textilien.

16. Verwendung der Verbindungen nach einem der Ansprüche 1 bis 12 für das Reinigen von harten Oberflächen.

17. Verwendung der Verbindungen nach einem der Ansprüche 1 bis 12 für das Reinigen und Waschen von Haut und Haar.

## Claims

1. Anionic amphiphilic compounds of the general formula I 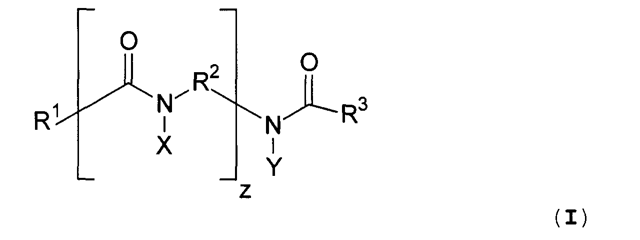 where
**R**^{**1**} and **R**^{**3**}**,** independently of one another, are a hydrocarbon radical having 1 to 22 carbon atoms,
**R**^{**2**} is a spacer,
**X** and **Y** represent independently of one another functional groups and
the degree of oligomerization **z** is from 1 to 10,
**X** and **Y** being independently of one another
- substituents according to formula XVI
-(C₂H₄O)_{α}(C₃H₆O)_{β}H **X(VI**)
where α = 0 to 50, β = 0 to 60, α + β = 1 to 100, wherein **R**^{**2**} is not C₂H₄ if β = 0,
- or substituents according to formula XVII
-(C₂H₄O)_{γ}(C₃H₆O)_{δ}-FR (**XVII**)
where γ = 0 to 20, δ = 0 to 20, and
γ + δ = 1 to 40,
and **FR** represents a functional radical -CH₂-COOM, -SO₃M, -PO(OM)₂, -C(O)-C₂H₃(SO₃M)-CO₂M', or -C₂H₄-SO₃M, wherein M, M' are alkali, ammonium, alkanol ammonium, or ½ alkaline earth metal, wherein the alkoxide units are incorporated randomly or blockwise and the sequence is optional.

2. The compounds according to claim 1, **characterized in that**
the hydrocarbon radicals **R**^{**1**} and **R**^{**3**} are unbranched or branched, saturated or unsaturated, and
the spacer **R**^{**2**} is an unbranched or branched chain comprising 2 to 100 carbon atoms, which comprises each 0 to 20 oxygen and nitrogen atoms, and 0 to 4 sulfur atoms, and 0 to 3 phosphorus atoms, and has 0 to 20 functional side groups.

3. The compounds according to any one of claims 1 or 2, **characterized in that**
the degree of oligomerization **z** is from 1 to 4.

4. The compounds according to any one of claims 1 to 3, **characterized in that**
the hydrocarbon radicals **R**^{**1**} and **R**^{**3**} in formula I comprise independently of one another 7 to 17 carbon atoms.

5. The compounds according to any one of claims 1 to 4, **characterized in that**
**R**^{**2**} is a spacer comprising as body
an unbranched or branched alkylene chain of formula II
-CₐH₂ₐ- (**II**)
where **a** = 2 to 18,
or an unbranched or branched alkenylene chain of formula III
-C_{b}H_{2b}-CH=CH-C_{c}H_{2c}- (**III**)
where **b** + **c** = 2 to 16, each **b** and **c** being greater than zero,
or an unbranched or branched alkynylene chain of formula IV
-C_{d}H_{2d}-C≡C-CₑH₂ₑ- (**IV**)
where **d** + **e** = 2 to 16, each **d** and **e** being greater than zero, and wherein the spacer in the bodies according to the formulas II to IV has 0 to 4 carbonyl-, amino-, or acylamino groups at any place of the chain.

6. The compounds according to any one of claims 1 to 4, **characterized in that**
**R**^{**2**} is a spacer comprised of alicycles according to formula V
-C_{f}H_{2f}-cycloC₆H₁₀-C_{g}H_{2g}- (**V**)
where each **f** and **g** = 1 to 6,
or according to formula VI
-3(4),8(9)-di(methylene)-tricyclo[5.2.1.0^{2.6}]decane- **(VI).**

7. The compounds according to any one of claims 1 to 4, **characterized in that**
**R**^{**2**} is a spacer comprised of unsubstituted or substituted aromatics according to formula VII
-CₕH₂ₕ-C₆R₄-(CᵢH₂ᵢ-C₆R₄)ⱼ₁-Cⱼ₂H₂ⱼ₂- (**VII**)
or according to formula VIII
-CₕH₂ₕ-C₁₀R₆-CⱼH₂ⱼ- (**VIII**)
where each **h, j, j1,** and **j2** = 0 to 8 and **i** = 1 to 8, and
**R** represents independently of one another H or C₁- to C₄-alkyl.

8. The compounds according to any one of claims 1 to 5, **characterized in that**
the spacer **R**^{**2**} bears amino-, acylamino-, carbonyl-, or carboxyl functions as functional side groups.

9. The compounds according to any one of claims 1 to 5, **characterized in that**
the spacer **R**^{**2**} comprises 0 to 20 oxygen and/or nitrogen atoms each, 0 to 4 sulfur atoms, and 0 to 3 phosphorus atoms, wherein at least one of the heteroatoms occurs at least once.

10. The compounds according to claim 9, **characterized in that**
**R**^{**2**} represents a spacer according to formula (IX)
-CₖH₂ₖ-CₓR_{y}-Z-CₓR_{y}-C₁H₂ₗ- (**IX**)
where each **k** and **1 =** 0 to 8, **x** = 6 and **y** = 4, or **x** = 10 and **y** = 6, or **x** = 14 and **y** = 8, and **Z** = O, CO, NH, NR¹, N-C(O)R¹,SO₂, wherein **R**^{**1**} represents a hydrocarbon radical having 1 to 22 carbon atoms, and **R** is independently of one another H or C₁- to C₄-alkyl.

11. The compounds according to claim 9, **characterized in that**
**R**^{**2**} represents a spacer according to formula X
-CₘH₂ₘ-(CₙH₂ₙO)ₚ-C_{q}H_{2q}- (**X**)
where **m** = 1 to 4, **n** = 2 to 4, **p** = 1 to 20, and **q** = 1 to 4, wherein also mixed alkoxide units may be present and the sequence of said alkoxide units can be optional.

12. The compounds according to claim 9, **characterized in that**
**R**^{**2**} represents a spacer according to formula XI
-CᵣH₂ᵣ(RNCₛH₂ₛ)ₜ-CᵤH₂ᵤ- (**XI**)
or according to formula XII
- [CᵣH₂ᵣ[RN-C(O)-NR]ₜ-CᵤH₂ᵤ]_{w}- (**XII**)
or according to formula XIII
-[CᵣH₂ᵣ[RNC(O)CᵥH₂ᵥC(O)NR]ₜ-CᵤH₂ᵤ]_{w}- (**XIII**)
or according to formula XIV
- [CᵣH₂ᵣ[RN-C(O)-CH=CH-C(O)-NR]ₜ-CᵤH₂ᵤ]_{w}- (**XIV**)
or according to formula XV
-[CᵣH₂ᵣ[RNC(O)CₓR_{y}C(O)NR]ₜ-CᵤH₂ᵤ]_{w}- (**XV**)
where **r** = 2 to 4, **s** = 2 to 4, **t** = 1 to 20, **u** = 2 to 4, **v** = 0 to 12, **w** = 1 to 6, **x** = 6 and **y** = 4, or **x** = 10 and **y** = 6, or **x** = 14 and **y** = 8, wherein **R** represents independently of one another H or C₁- to C₄-alkyl.

13. Use of the compounds according to any one of claims 1 to 12 as emulsifiers or demulsifiers.

14. Use of the compounds according to any one of claims 1 to 12 as auxiliaries in metal working, ore mining, or surface finishing.

15. Use of the compounds according to any one of claims 1 to 12 as textile auxiliaries or for cleaning and washing textiles.

16. Use of the compounds according to any one of claims 1 to 12 for cleaning hard surfaces.

17. Use of the compounds according to any one of claims 1 to 12 for cleaning and washing skin and hair.

## Revendications

1. Composés amphiphiles anioniques de formule I : 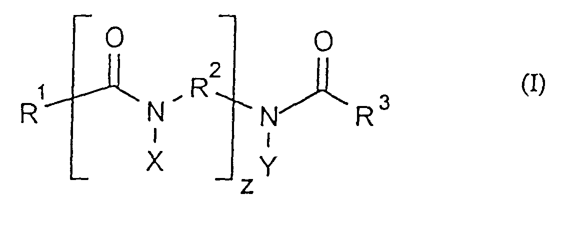 dans laquelle :
R¹ et R³ désignent, indépendamment l'un de l'autre, un radical hydrocarboné avec 1 à 22 atomes de carbone,
R² est un espaceur,
X et Y désignant indépendamment l'un de l'autre des groupements fonctionnels et le degré d'oligomérisation z est de 1 à 10,
X et Y désignant indépendamment l'un de l'autre:
- des substituants de formule XVI :
- (C₂H₄O)_{α}(C₃H₆O)_{β}H (XVI)
avec α =0 à 50, β = 0 à 60, α + β = 1 à 100, R² n'étant pas C₂H₄ lorsque β = 0,
- ou des substituants de formule XVII :
-(C₂H₄O)_{γ}(C₃H₆O)δ-FR (XVII)
avec à chaque fois γ = 0 à 20, δ = 0 à 20, et γ + δ = 1 à 40,
FR désignant un radical fonctionnel -CH₂-COOM,-SO₃M, -PO(OM)₂, -C(O)-C₂H₃(SO₃M)-CO₂M' ou -C₂H₄-SO₃M, avec M,M' = métal alcalin, ammonium, alcanolammonium ou ½ métal alcalino-terreux,
les unités alcoxydes étant liées de façon statistique ou séquencée et la séquence étant quelconque.

2. Composés selon la revendication 1, **caractérisés en ce que** :
les radicaux hydrocarbonés R¹ et R³ sont non ramifiés ou ramifiés, saturés ou insaturés, et
l'espaceur R² désigne une chaîne non ramifiée ou ramifiée avec 2 à 100 atomes de carbone, qui présente 0 à 20 atomes d'oxygène et d'azote et 0 à 4 atomes de soufre ainsi que 0 à 3 atomes de phosphore et présente 0 à 20 groupes latéraux fonctionnels.

3. Composés selon l'une quelconque des revendications 1 et 2, **caractérisés en ce que** le degré d'oligomérisation Z est de 1 à 4.

4. Composés selon l'une quelconque des revendications 1 à 3, **caractérisés en ce que** les radicaux hydrocarbonés R¹ et R³ de la formule I contiennent indépendamment l'un de l'autre 7 à 17 atomes de carbone.

5. Composés selon l'une quelconque des revendications 1 à 4, **caractérisés en ce que** R² désigne un espaceur qui contient comme corps de base une chaîne alkylène non ramifiée ou ramifiée de formule II :
-CₐH₂ₐ- (II)
avec a = 2 à 18,
ou une chaîne alkylène non ramifiée ou ramifiée de formule III
-C_{b}H_{2b}-CH=CH-C_{c}H_{2c}- (III)
avec b + c = 2 à 16, b et c étant à chaque fois supérieurs à 0,
ou une chaîne alcynylène non ramifiée ou ramifiée de formule IV :
-C_{d}H_{2d}-C ≡ C-CₑH₂ₑ₋ (IV)
avec d + e = 2 à 16, d et e étant à chaque fois supérieurs à 0, et les corps de base de formules II à IV de l'espaceur présentant en un point quelconque de la chaîne 0 à 4 groupements carbonyle, amino ou acylamino.

6. Composés selon l'une quelconque des revendications 1 à 4, **caractérisés en ce que** R² signifie un espaceur qui est constitué d'alicyclène de formule V :
-C_{f}H_{2f}-cyclo C₆H₁₀-C_{g}H_{2g}- (V)
avec f et g = à chaque fois 1 à 6,
ou de formule VI :
-3(4),8(9)-di(méthylène)tricyclo[5.2.1.0^{2,6}]décane- (VI)

7. Composés selon l'une quelconque des revendications 1 à 4, **caractérisés en ce que** R² désigne un espaceur qui est constitué d'aromates non substitués ou substitués de formule VII :
-CₕH₂ₕ-C₆R₄-(CᵢH₂ᵢ-C₆R₄)ⱼ₁-Cⱼ₂H₂ⱼ₂- (VII)
ou de formule VIII :
-CₕH₂ₕ-C₁₀R₆-CⱼH₂ⱼ- (VIII)
avec h, j, j1 et j2 = à chaque fois O à 8 et i = 1 à 8, et
avec R, qui désigne indépendamment l'un de l'autre à chaque fois H ou un alkyle en C₁-C₄.

8. Composés selon l'une quelconque des revendications 1 à 5, **caractérisés en ce que** l'espaceur R² porte comme groupements latéraux fonctionnels des fonctions amino, acylamino, carbonyle ou carboxyle.

9. Composés selon l'une quelconque des revendications 1 à 5, **caractérisés en ce que** l'espaceur R² contient à chaque fois 0 à 20 atomes d'oxygène et/ou d'azote, 0 à 4 atomes de soufre et 0 à 3 atomes de phosphore, au moins l'un des hétéroatomes étant présent au moins une fois.

10. Composés selon la revendication 9, **caractérisés en ce que** R² désigne un espaceur de formule (IX) :
-CₖH₂ₖ-CₓR_{y} -Z-CₓR_{y}-C₁H₂₁- (IX)
avec k et l à chaque fois égaux à 0 à 8, x = 6, y = 4 ou x = 10 et y = 6 ou x = 14 et y = 8, Z = 0, CO, NH, NR¹, N-C(O)R¹, SO₂, R¹ signifiant un radical hydrocarboné avec 1 à 22 atomes de carbone et R étant indépendamment l'un de l'autre à chaque fois H ou un alkyle en C₁-C₄.

11. Composés selon la revendication 9, **caractérisés en ce que** R² désigne un espaceur de formule X :
-CₘH₂ₘ-(CₙH₂ₙO)ₚ-C_{q}H_{2q}- (X)
avec m = 1 à 4, n = 2 à 4, p = 1 à 20 et q = 1 à 4, des unités alcoxyde mixtes pouvant également être présentes et la séquence des unités alcoxyde étant quelconque.

12. Composés selon la revendication 9, **caractérisés en ce que** R² désigne un espaceur de formule XI :
-CᵣH₂ᵣ(RNCₛH₂ₛ)ₜ-CᵤH₂ᵤ- (Xi)
ou de formule XII :
- [CᵣH₂ᵣ[RN-C(O)-NR]ₜ-CᵤH₂ᵤ]_{w}- (XII)
ou de formule XIII :
-[CᵣH₂ᵣ[RNC(O)CᵥH₂ᵥC(O)NR]ₜ-CᵤH₂ᵤ]_{w}- (XIII)
ou de formule XIV :
-[CᵣH₂ᵣ[RN-C(O)-CH=CH-C(O)-NR]ₜ-CᵤH₂ᵤ]_{w}- (XIV)
ou de formule XV :
- [CᵣH₂ᵣ[RNC(O)CₓR_{y}C(O)NR]ₜ-CᵤH₂ᵤ]_{w}- (XV)
avec r = 2 à 4, s = 2 à 4, t = 1 à 20, u = 2 à 4, v = 0 à 12, w = 1 à 6, x = 6 et y = 4 ou x = 10 et y = 6, ou x = 14 et y = 8, les groupements R désignant indépendamment l'un de l'autre à chaque fois H ou un alkyle en C₁-C₄.

13. Utilisation des composés selon l'une quelconque des revendications 1 à 12 comme émulsifiants ou désémulsifiants.

14. Utilisation des composés selon l'une quelconque des revendications 1 à 12 comme agents auxiliaires pour l'usinage de métaux, l'extraction de minerais ou la finition de surface.

15. Utilisation des composés selon l'une quelconque des revendications 1 à 12 comme agents auxiliaires textiles ou pour le nettoyage et le lavage de textiles.

16. Utilisation des composés selon l'une quelconque des revendications 1 à 12 pour le nettoyage de surfaces dures.

17. Utilisation des composés selon l'une quelconque des revendications 1 à 12 pour le nettoyage et le lavage de la peau et des cheveux.
